# EUROPEAN PATENT APPLICATION

(11) **EP 1 310 258 A1**
(43) Date of publication of application: **14.05.2003**
(21) Application number: 01402868.2
(22) Date of filing: 08.11.2001
(51) Int. Cl.: A61K 31/5685, A61K 31/57, A61P 25/28

(54) **Enantiomers of steroids for the enhancement of memory and cognitive function**

(71) Applicant: Baulieu, Etienne-Emile, 92200 Neuilly-sur-Seine (FR); Akwa, Yvette, 75018 Paris (FR)
(72) Inventor: Baulieu, Etienne-Emile, 92200 Neuilly-sur-Seine (FR); Akwa, Yvette, 75018 Paris (FR)
(74) Representative: Warcoin, Jacques

(57) **Abstract**

The present invention relates to the use of enantiomers of steroids in the restoration of memory loss in humans. The present methods may be used to enhance memory or cognitive function in patients suffering from age-related decline in memory function, from neurological or neuropsychiatric disorders such as cognitive deficits, amnesia or dementia, or from neurodegenerative diseases such as Alzheimer's Disease. Steroids include, but are not limited to, neurosteroids such as dehydroepiandrosterone sulfate and structurally related molecules. Of particular interest are steroid enantiomers that are not metabolized by steroid enzymes such as sulfatases, as well as those that do not activate steroid hormone receptors. Such enantiomers possess potentially higher therapeutic efficacy without the classical hormonal effects attributable to these receptors.

## Description

The present invention relates to the use of enantiomers of steroids in the restoration of memory loss in humans. The present methods may be used to enhance memory or cognitive function in patients suffering from age-related decline in memory function, from neurological or neuropsychiatric disorders such as cognitive deficits, amnesia or dementia, or from neurodegenerative diseases such as Alzheimer's Disease. Steroids include, but are not limited to, neurosteroids such as dehydroepiandrosterone sulfate and structurally related molecules. Of particular interest are steroid enantiomers that are not metabolized by steroid enzymes such as sulfatases, as well as those that do not activate steroid hormone receptors. Such enantiomers possess potentially higher therapeutic efficacy without the classical hormonal effects attributable to these receptors.

### Background of the invention

Steroids include neurosteroids which are synthesized in the nervous system. The presence of neurosteroids has been observed in the brains of various mammals, including rat, mouse, monkey (Baulieu, 1981, In *Steroid Hormone Regulation in the Brain,* Fuxe K, Gustafsson JA, Wetterberg L eds, pp 3-14, Pergamon Press,; Corpéchot, 1981, *Proc Natl Acad Sci USA*, 78: 4704-4707; Corpéchot, 1983, *Brain Res.,* 270: 119-125; Robel, 1986, In *Neuroendocrine Molecular Biology,* Fink; G., Hamar AJ, McKerns, KW, eds., pp. 367-377; Robel, 1999, In *Neurosteroids: A new regulatory function in the nervous system,* Baulieu E.E., Robel P., Schumacher M. eds, pp 1-25, Humana Press); and human (Hammond, 1983, *J. Steroid Biochem.,* 18:185-9; Lanthier, 1986, J Steroid Biochem. 25:445-9; Lacroix, 1987, *J Steroid Biochem.,* 28:317-25; Bixo, 1995, *J Steroid Biochem. Mol. Biol.* 55:297-303; Bixo, 1997, *Brain Res.* 1997,764:173-8). Neurosteroids act within the central nervous system through paracrine or autocrine mechanisms and regulate several brain functions including neurotransmission and behavior (Baulieu, 1997, *Recent Prog. Horm. Res*.,52:1-32; Akwa & Baulieu, 1999, *J Soc Biol.,* 193:293-8; Schumacher et al., 2000, *J Neurocytol*., 29:307-26).

Certain neurosteroids have been observed to have memory-enhancing activity. For example, dehydroepiandrosterone sulfate (DHEAS) and pregnenolone sulfate (PREGS) have been shown to improve memory performance in various assays, including by reducing amnesia in rodents induced by agents such as dimethylsulfoxide, ethanol, anticholinergics and excitory amino acid receptor antagonists (Flood & Roberts., 1988, *Brain Res*., 10:448:178-81;. Flood et al., *Brain Res.,* 447:269-78; Flood et al., 1992, *Proc.Natl.Acad.Sci. USA,* 89:1567-71; Flood et al., 1995, *Proc.Natl.Acad.Sci.USA,* 92:10806-10; Mayo et al., 1993, *Brain Res.,* 607:324-8; Melchior & Ritzman, 1996, *Pharmacol. Biochem. Behav*., 1996 Jan;53(1):51-6.Meziane et al., 1996, *Psychopharmacology (Berl)*, 126:323-30; Vallée et al., 1997, *Proc.Natl.Acad.Sci. USA*, 94:14865-70; Reddy & Kulkarni, 1998, *Brain Res*.,791:108-16; Dardaunéry et al., 2000, *Brain Res*., 852:173-9; Ladurelle et al., 2000, *Brain Res.,* 858:371-9). In addition, pharmacological studies have shown that DHEAS and PREGS are able to improve age-related memory impairment in rodents (Flood and Roberts, 1988, *Brain Res.,* 448:178-81; Reddy & Kulkarni, 1998, *Brain Res.,* 799:215-29.; Vallée, 1997, *Proc.Natl.Acad.Sci. USA,* 94:14865-70).

It is thought that the promnestic action of neurosteroids occurs by regulating neurotransmitter receptor function (particularly gamma-amino-butyric acid type A (GABA_{A}) and N-methyl-D-aspartate (NMDA) receptor activities) in several brain regions (Mayo et al., 1993, *Brain Res.,* 607:324-8; Mathis et al., 1994, *Psychopharmacology (Berl)*, 116:201-6; Cheney et al., *1995, Neuroreport,* 6:1697-1700; Flood et al., 1995, *Proc.Natl.Acad.Sci.USA*, 92:10806-10; Mathis et al., 1996, *Neuropharmacology,* 35:1057-64; Meziane et al., 1996, *Psychopharmacology (Berl),* 126:323-30; Maurice et al., 1997, *Behav Brain Res.* 1997, 83:159-64; Reddy & Kulkarni, 1998, *Brain Res.,* 799:215-29; Pallarès et al., 1998, *Neuroscience*, 87:551-8).

The structural features of steroids are crucial for their biological activity, and various studies have demonstrated a marked stereoselectivity, particulary diastereoselectivity, for steroid regulation of neurotransmitter receptor function (Paul & Purdy, 1992, *FASEB J.,* 6:2311-22; Lambert 1995, *Cell Mol Neurobiol.,* 16:155-74; 1999; Lambert 2001, *Int Rev Neurobiol.,* 46:177-205). Diastereomers are compounds with mutiple chiral centers that differ in configuration at one or some, but not all, chiral centers. Stereoisomers in which the absolute configuration of all chiral centers is inverted are called enantiomers. As opposed to diastereomers, enantiomers have identical physical properties, but differ in shape (hence have different rotation of plane-polarized light) and act dissimilarly only in a chiral environment (e.g., dissimilar binding to protein receptor sites). Recent studies have provided evidence for enantioselectivity of steroid-induced GABA_{A} receptor activity (Covey et al., 2000, *J. Pharmacol. Exter.,* 293, 1009-16; Wittmer et al., 1996, *Mol. Pharmacol*., 50, 1581-6).

Dementia is a worldwide problem which can be grouped into two categories: i) dementia that presents without prominent motor signs, including Alzheimer's disease, frontotemporal dementia and prion diseases ; and ii) dementia characterized at onset by prominent motor signs, including vascular dementia, dementias with Lewy bodies, progressive supranuclear palsy, cortico-basal ganglionic degeneration, disease. In North America and Western Europe, as the population continue to age rapidly, the problem of age-related dementia is expected to become enormous. It is estimated that approximately 70 percent of all cases of dementia (Paquid; USA) are caused by Alzheimer disease (with 10-15 patients per 10,000 individuals per year in Europe), and a further 15 percent are vascular dementia. At the end stage of either Alzheimer's disease or vascular dementia, memory and cognitive function are largely lost and the individual becomes completely dependent for all basic function.

In addition to dementia, there are many other common causes of memory loss and loss of cognitive function, such as injury, neurological conditions, neurodegenerative diseases, and psychiatric disorders.

Despite the frequency and debilitating nature of memory loss and loss of cognitive function, there is a striking dearth of effective and safe treatments for these conditions. For example, there are currently no recognized or licensed treatment for late stage dementia, and the several treatments that are available for mild to moderate Alzheimer's disease, such as cholinesterase inhibitors and NMDA receptor-blocking antagonists, have significant side effects that necessitate their administration under close medical supervision.

Clearly, new, effective, and safe treatments are acutely needed for dementia and other forms of memory loss and loss of cognitive function. The present invention addresses these and other needs.

### Summary of the Invention

The present invention provides novel methods for the enhancement of memory and cognitive function in mammals, particularly humans. These methods are based on the surprising discovery that the administration of steroid enantiomers results in striking improvements in memory and cognitive function.

Accordingly, in one aspect, the present invention provides a method for enhancing memory in an individual, the method comprising administering to the individual a therapeutically-effective amount of an enantiomer of a steroid.

In another aspect, the present invention provides a pharmaceutical composition, said composition comprising an enantiomer of a steroid and a pharmaceutically acceptable carrier.

In another aspect, the present invention provides the use of an enantiomer of a steroid for the preparation of a medicament that is effective in the restoration of memory loss in a human.

In one embodiment, the individual has suffered memory loss resulting from a cause selected from the group consisting of age, neurological disorders, neuropsychiatric disorders, neurodegenerative disorders, and injury.

In another embodiment, the steroid is a neurosteroid. In another embodiment, the neurosteroid is DHEA sulfate. In another embodiment, the steroid is a steroid hormone. In another embodiment, the steroid is modified at least at one of the chiral or non chiral centers. In another embodiment, the steroid is modified by hydoxylation, esterification, etherification, reduction, oxididation, or substitution with an halogen or an alkyl group.

### Detailed description of the invention

The present invention provides novel methods and compositions for enhancing memory and cognitive function in mammals, particularly humans. Specifically, the present invention is based upon the surprising discovery that the administration of the enantiomers of steroids to mammals results in dramatic and unexpected improvements in their memory and cognitive performance. Such methods may be used to restore memory or cognitive function in individuals that have lost memory capacity or cognitive function due to any cause, including age, neurological or neurodegenerative diseases, psychiatric conditions, or injury. The enantiomer of any steroid may be used, in particular the non-naturally occurring enantiomer of a naturally-occurring steroid, preferably a neurosteroid.

### Steroid enantiomers

Any of a large variety of steroid enantiomers may be used to carry out the present methods. The steroids may be naturally-occurring, endogenous steroids, or may be non-natural, synthetic steroids, in particular a synthetic derivative of an endogenous steroid. In a preferred embodiment, the steroid is a neurosteroid, such as a 3 beta-hydroxy-delta 5-compound, including pregnenolone (PREG) or dehydroepiandrosterone (DHEA). Also preferred are sulfate esters of the steroids, e.g. PREGS or DHEAS. Also preferred are derivatives of PREG, DHEA, PREGS, or DHEAS, e.g. an enantiomer of any of these steroids (or any of the herein-described steroids) that contains a single substitution or modification (e.g., hydoxylation, esterification, etherification, reduction, oxidation, or substitution with a halogen or alkyl group), at any position. In another embodiment, any one of the specific steroid enantiomers may be excluded from the invention, such as the enantiomer of PREGS. For example, in one embodiment a method of enhancing memory or cognitive function is provided, comprising administering to an individual an enantiomer of a steroid other than PREGS Other suitable enantiomer steroids are enantiomers of any of the following steroids: 3 beta-hydroxy-androst-5-ene, androst-5-ene-3 beta,17 beta-diol, 3 beta,7 beta-dihydroxy-androst-5-ene-17-one, 3 beta,7 beta-dihydroxy-androst-5-ene-17-one; androst-5-ene-3 beta,7 alpha,17 beta-triol, 3 beta-hydroxy-androst-4-ene-17-one and the respective 3-sulfate esters; DHEA-tosylate, 3 betamethyl-androst-5-ene-17-one, 3 beta-methoxy-androst-5-ene-17-one, 6 alphamethyl-androst-5-ene-3 beta,17 beta-diol, 3 beta-hydoxy-pregn-5-ene, 17 alphahydroxy-PREG, pregn-5-ene-3 beta,20alpha-diol, 3beta,7 alpha-dihydroxy-pregn-5-ene-20-one, 3 beta-hydoxy-5,14-pregnadiene-20-one, 3 beta-hydoxy-16-dehydropregn-5-ene-20-one, 3 beta-hydoxy-B-Norpregn-5-ene-20-one, 3 beta-hydroxypregn-4-ene-20-one, pregn-4-ene-3 beta,20 alpha-diol, 3 beta,6 beta-dihydroxypregn-4-ene-20-one and their respective 3-sulfate esters, PREG-tosylate, 3 betamethyl-pregn-5-ene-20-one, 3 beta-methoxy-pregn-5-ene-20-one, 3 beta-hydroxy-5 alpha-pregnane-20-one, 3 beta-hydroxy-5 alpha-androstane-17-one 6 alpha-hydroxycortisol, 3,11 beta,17 alpha,21-tetrahydroxy-1,3,5(10)-19-Norpregnatriene-20-one, 3 beta,11 beta,17 alpha,21-tetrahydroxy-pregn-5-ene-20-one, 3 beta,11 beta,21-trihydroxy-pregn-5-ene-20-one and their respective 3-sulfate esters, 3 beta-methoxy-5 alpha-pregnane-20-one, and 3 beta-methoxy-5 alpha-androstane-17-one.

In specific embodiments of this invention, the steroid enantiomer is an enantiomer of an endogenous steroid hormone such as progesterone, testosterone, estradiol, cortisol, corticosterone, or aldosterone.

The present enantiomers may also represent modified or derivative forms of any of the herein-described steroids. Among the suitable modifications, which may be present alone or in any combination, include hydoxylation, esterification, etherification (particularly substituted with a methoxy group), reduction, oxidation, substitution with an halogen (fluor, chlore, brome and iode) or a straight-chain or branched-chain alkyl (particularly methyl or methoxy) group. In a preferred embodiment of this invention, the steroid modification occurs at least at one of the chiral centers of the above-said steroids, particularly by hydroxylation or sulfation.

Without being bound by the following theory, it is believed that one reason why the present steroid enantiomers are more active than the steroids themselves is because the enantiomers are not metabolized as efficiently as the steroid forms, and are thus present in an active form for a longer period of time. Accordingly, in a preferred embodiment, the present invention uses an enantiomer of a steroid wherein the enantiomer is metabolized less efficiently than the steroid counterpart. Similarly, the present invention also provides a method of evaluating an enantiomer of a steroid for the enhancement of memory or cognition in a patient, the method comprising administering the enantiomer and its steroid counterpart to a non-human animal or to a cell, and comparing the relative metabolism of each compound in the animal or cell, wherein a finding that the enantiomer is metabolized less efficiently than its steroid counterpart indicates that the enantiomer is likely to be useful in the enhancement of memory or cognition.

Any suitable method, including in vitro and in vivo methods, may be used to assess the level of metabolism of the steroid and the enantiomer. In one typical embodiment, an equivalent amount of each of the compounds is administered, e.g. by injection, into two sets of animals, and the levels of each of the compounds is detected, e.g. using mass spectrometry, after a given period of time. In such embodiments, if the level of the enantiomer is higher than the level of the steroid after the period of time, then it is concluded that the enantiomer is metabolized less efficiently than the steroid. In one embodiment, the metabolism of the enantiomers or steroids by sulfatases is assessed. In another embodiment, the level of metabolism in the brain or the liver is assessed in vivo.

In addition, and also without being bound by the present theory, it is believed that the enantiomers enhance memory and cognitive function at least in part by a different mechanism than that used by the steroid forms. In particular, it is believed that the steroid forms enhance memory and cognitive function primarily through their interaction with neurotransmitter receptors, such as the NMDA receptor, whereas the enantiomers affect memory and cognition largely through a neurotransmitter receptor-independent mechanism. Accordingly, in one embodiment, the enantiomer used in the present invention interacts less well with one or more neurotransmitter receptors than does its steroid counterpart. Similarly, the present method provides a method of evaluating an enantiomer for use in the enhancement of memory or cognition in a patient, the method comprising detecting the interaction of the enantiomer and its steroid counterpart with one or more neurotransmitter receptors, wherein a finding that the enantiomer interacts less well with one or more receptor than its steroid counterpart indicates that the enantiomer is likely to be useful for the enhancement of memory or cognition.

Any suitable method can be used to assess the interaction of the enantiomers and the steroids with a neurotransmitter receptor, including in vitro or in vivo assays for binding of the compounds to the receptor or for effects of the compounds on the activity of the receptors.

The herein-described enantiomers may be prepared using any standard method, including by one or several enzymatic or chemical reactions. In a preferred embodiment, the enantiomers are prepared using methods described by Nilsson et al. (1998; *J. Med. Chem*., 41, 2604-1).

The suitability of any given steroid enantiomer may be assessed using any in vivo assay for memory or cognitive performance in any animal model, in particular mice or rats. In one embodiment, the suitability of an enantiomer is assessed by testing the effect of the enantiomer in a short-term memory assay such as a spatial two-trial recognition test, e.g., in a Y-maze, or in a long-term memory assay such as the water maze and passive avoidance tests.

### Memory and cognitive function

The present invention may be used to enhance memory or cognitive function in any mammal, preferably humans. Preferably, the methods are used to enhance memory or cognitive function lost as a result of any of a number of diseases or conditions, but the methods may also be used to enhance memory or cognition even in a healthy individual who has not suffered from memory loss or a loss of cognitive function.

The loss of memory or cognitive function may be the result of any disease or condition, including, but not limited to, normal age-related memory loss or dementia, neurodegenerative disorders such as Alzheimer's Disease, psychiatric disorders such as anxiety, chronic stress, depression and sleep disturbance, drug-related memory loss, or amnesia resulting, e.g. from an injury or other trauma.

### Pharmaceutical compositions and administration

In one embodiment of this invention, enantiomers of steroids are used for the preparation of a pharmaceutical composition that is effective for the improvement of memory or cognitive performance in a human subject under non-pathological conditions.

In another embodiment, enantiomers of steroids are used for the preparation of a pharmaceutical composition that is effective for the treatment of memory or cognitive defects associated with a neurological or neuropsychiatric disorder or diseases, preferably, cognitive deficits, amnesia, dementia and neurodegenerative diseases, such as Alzheimer's Disease.

Accordingly, the present invention also provides pharmaceutical compositions comprising any of the herein-described steroid enantiomers and a pharmaceutically acceptable carrier or excipient. Such compositions may be prepared for any form of administration, including oral (e.g., in the form of tablets, capsules, lozenges, chewing gum, troches, powders, syrups, elixirs, aqueous solutions and suspensions), topical (e.g., including the use of a patch or other transdermal delivery device), or parenterally. Such compositions may include any standard carrier or excipient, and may be performed using any of a large number of well known methods. Suitable methods and compositions for pharmaceutical compositions and administration are taught, e.g., in Remington's Pharmaceutical Sciences (1995. Mack Publishing Co.: Easton, PA).

The present compounds and pharmaceutical compositions may be administered in any standard delivery method, including, but not limited to, oral, transdermal, parenteral, and topical. Administration can be by means of a pump for periodic or continuous delivery.

The present compounds and pharmaceutical compositions may be administered at any of a range of effective doses. The particular dose adminstered will likely be determined by a qualified medical practitioner, and will include considerations concerning the patient's age, weight, medical condition, medical history, other medications, etc. Typically, the dose administered will fall in the range of from about 0.1 mg to about 5.0 mg/kg, preferably about 1 mg/kg.

The present compounds may be administered alone or in combination with any other compound or therapeutic regimen, including in combination with other steroid enantiomers or with steroids.

In a preferred embodiment of the invention, the subject is a human subject. The subject may be of any age. In one embodiment, the subject is an aged person, e.g. 50 years-old or older.

The efficacy of any of the herein-described compounds on a patient may be assessed in any way, including by the subjective opinion of a medical practitioner, as well as by more quantitative methods, e.g., in human routine diagnostic tests for memory, cognition and dementia include mental status screening by the Mini Mental State examination (MMSE) and neuropsychological testing such as the Clinical Dementing Rating Score (CDR) tests (Folstein, 1975, *J. Psychiatr. Res.,* 12:189-98; Hughes, 1982, *Br. J. Psychiatry.* 140:566-72.), and the Alzheimer Disease Assessement Scale-cognitive (ADAS-cog). Diagnostic criteria may be those proposed by the National Institute of Neurological and Communicative Disorders and Stroke - Alzheimer's Disease and Related Disorders Association (NINCDS-ADRDA) (American Psychiatric Association, 1987; McKhann, 1984, *Neurology*., 34:939-44.).

### Examples

The following examples illustrate the present invention, but of course, should not be construed as in any way limiting its scope.

### Example 1 : Experimental Protocol

Male Sprague-Dawley rats and Swiss mice (Janvier, Le Genest St-Isle, France) 2 months of age and weighing 250-300g for rats and 30-32g for mice at the time of surgery were used. They were grouped-housed in a temperature (22°C ± 1°C) and humidity (50% ± 5%) controlled room. They were maintained on a 12-h light/12-h dark (08.00-20.00) schedule and had access to food and water *ad libitum.*

Rats were anaesthesized with a mixture of 5% ketamine (Imalgène 1000, Merial, France) and 0.1% acepromazine (Vetranquil 1%,) in saline, administered i.p. at 1.5 ml/kg. Mice were anesthetized with a mixture of 0.5% ketamine and 0.1% xylazine, under the volume of 15 ml/kg.

Animals were mounted in a stereotaxic and implanted with unilateral stainless steel cannula guides (23-gauge, 7 mm long) above the lateral ventricle, according to the coordinates from the atlas of rats and mice. Cannulas were secured to the skull with stainless steel screws and dental cement.

Animals were used for experiments after a 7-day postrecovery period. Each animal was handled daily for 3 days before the experiments and on the day before each experiment, cannula function was confirmed by gravity flow of isotonic saline.

### Example 2 : Treatment and Injection Procedures

PREGS was obtained from Steraloïds (Newport, RI, Etats-Unis); the steroid enantiomers *ent*-SPREG et *ent*-SDHEA were synthesized as previously described (Nilsson *et al*., 1998, *op. cit*.). All compounds were dissolved in 0.30 % saline (vehicle)

PREGS was administered iCV at doses of 0 (vehicle), 0.05, 0.25, 0.5, 2.5, 5.0 and 12.5 nmol in rats and of 0 (vehicle), 0.05, 0.25, 0.5, 2.5, 5.0 and 12.5 nmol mice

*ent*-PREGS was injected ICV at doses of 0 (vehicle), 0.025, 0.05, 0.25, 0.5 nmol in rats and 0 (vehicle), 0.025, 0.05, 0.25, 0.5 and 2.5 nmol in mice.

*ent*-SDHEA was administered iCV at doses of 0 ; 0,25 ; 0,25 nmol in rats.

Drug and control-vehicle solutions were injected unilaterally through the cannula guide. A total volume of 5 µl in rats and 2.5 µl in mice was infused using a KDS pump (Fisher Scientific), at a constant rate of 1µl/ min, through a 30 gauge stainless steel injector attached to a 10 µl microsyringe (Hamilton) by polyethylene tubing. The injectors were 1.5 mm and 0.8 mm longer in rats and mice, respectively, than the cannula guides and were left in place an additional 40 s to allow diffusion of solutions away from the tip.

### Example 3 : Behavioral Testing

The two-trial memory task based on spontaneous exploration of novelty was adapted from the ones described by Dellu et al. (1992, *Brain Res*., 588, 132-9) in rats and by Ladurelle et al. (2000, *Brain Res*., 858, 371-9) in mice.

Experiments were performed in a gray polyvinychloride Y-maze. The size of each arm was 40 cm long, 15 cm wide and 35 cm high for rats, and 30 cm long, 10 cm wide and 17 cm high for mice. The floor of the maze was covered with odor-saturated sawdust which was mixed after each trial. The maze was placed in a sound-attenuated room under dim illumination. Numerous visual cues were placed on the walls of the testing room and kept constant during the entire behavioral testing..

The test consisted of two trials, separated by a 6-h intertrial interval. During the first trial (acquisition phase), one arm of the Y-maze (subsequently called novel arm) was closed with a guillotine door, thus allowing the animal to explore only the other two arms, during 5 min and 3 min for rats and mice, respectively. During the second trial (retention phase), the animals had access to the three arms for 5 min. The time spent in each arm of the maze was recorded manually every minute. Recognition was assumed to have occurred when the animal spent more time in the novel arm compared with the familiar ones. The total number of arm visits provided a measure of general motor activity.

Drugs were administered immediately after the acquisition phase which thus was not affected, and retention was then tested following a long inter-trial interval , the time at which vehicle-control animals did not show any discrimination in the exploration duration of the three arms of the Y-maze (chance level is 33 %).

The influence of steroids on retention performance are thus interpreted as being the result of changes in memory processing and neurotransmitter receptor activity occurring shortly after acquisition and during the consolidation phase.

### Example 4 :Analysis of the results

A between-subjects design was used where each observation was made for a separate animal. Data were reported as the amount of time spent in the novel arm relative to the total duration of visits in the three arms (novel/total x 100) during the first 3 min.

They were expressed as mean ± SEM and analyzed by using one-way ANOVA followed by Newmann-Keuls posthoc test. Significance was defined as P<0.05 or P<0.01.

Studies required 2-3 experiments. The data were pooled between the different experiments because the variation was low for the values of control or same-drug doses-treated animals.

### Example 5: Spatial Memory Performances of Rats and Mice After ICV Administration of PREGS.

The effect of the neurosteroid PREGS on spatial memory recognition was analyzed by using several doses of the steroid (0.025-12.5 nmol, ICV) and U-inverted dose-response curves were obtained in both rats and mice.

Overall group comparison indicated that PREGS had a significant treatment effect in the two species [one-way ANOVA, F(6,56) = 6.34; P = 0.0001 for rats and F(7,53) = 3.66; P = 0.0027 for mice].

Detailed analysis using the Newman-Keuls test revealed that the % time spent in the novel arm was significantly higher in the steroid-injected groups at the PREGS-doses of 0.5 nmol in rats and 5.0 nmol in mice relative to vehicle-control groups (P< 0.05).

Thus, these data show that the natural PREGS directly improves memory performance.

### Example 6: Spatial Memory Performances of Rats and Mice After ICV Administration of ent-PREGS

The effect of the synthetic enantiomer of PREGS (*ent*-PREGS) on spatial memory retention was examined at doses of 0.025-2.5 nmol, ICV.

*ent*-PREGS was found to affect spatial recognition performances of rats and mice, in a dose-dependent and bell-shaped manner.

One-way ANOVA showed a significant effect of treatment, F(4,52) = 6.34, P = 0.0073 for rats and F(6,38) = 4.97, P = 0.0008 for mice.

Post-hoc Newman-Keuls test indicated that performance was significantly increased in groups given *ent*-PREGS at the doses of 0.05 nmol in rats (P<0.05) and 0.5 nmol in mice (P< 0.01) compared to vehicle-control groups.

Thus, these results demonstrate that *ent*-PREGS is not only very active upon memory performances, but it is even more active than PREGS.

### Example 7: Spatial Memory Performances of Rats and Mice After ICV Administration of ent-DHEAS

The effect of the synthetic enantiomer of DHEAS (*ent*-DHEAS) on spatial memory retention was examined at doses of 0.025 et 0.25 nmol ICV.

*ent*-SDHEA significantly increased memory performances in rats at the doses of 0,025 nmol (P<0.05)

Thus, ent-SDHEA is very active on memory performance even so more than *ent*-PREGS and PREGS.

### Example 8 : Coadministration of Steroids with the competitive and selective NMDA receptor antagonist AP5 and Cognitive Performance

The effects of the ICV injection of AP5, at ICV doses of 2 and 10 nmol in mice and rats, respectively, was studied on the memory-enhancing effects of PREGS (0.5 nmol in rats and 5.0 nmol in mice) and ent-PREGS (0.05 nmol in rats and 5.0 nmol in mice).

Comparison between the recognition performances of all drug-treated groups (AP5, PREGS, *ent*-PREGS, PREGS + AP5, *ent*-PREGS + AP5) revealed a significant effect of treatment [F(5,71) = 9,39; P = 0.0001 in rats; F(5,57) = 6,14; P = 0.0001 in mice].

Detailed post-hoc Newman-Keuls test analysis indicated that performance did not significantly differ between vehicle-control and AP5-treated groups of rats and mice (P > 0.05).

As observed previously (Examples 5 & 6), the ICV effective doses of PREGS and *ent*-PREGS alone significantly increased recognition performances in rats and mice compared with vehicle-controls (P < 0.05).

When AP5 (rat: 10 nmol; mice: 2 nmol) was administered together with PREGS, the memory performances were significantly reduced in rats as well as in mice compared to the groups treated with the neurosteroid alone (P < 0.05).

However, the animals coinjected with *ent*-PREGS and AP5 did not perform differently from those receiving *ent*-PREGS alone (P > 0.05).

Thus, enantioselectivity is demonstrated *in vivo* at the NMDA receptors suggesting that *ent*-PREGS and PREGS act by different mechanisms.

Besides different affinities for NMDA the receptors, the larger effect of ent-PREGS on memory may result as well from another mechanism related to differences in brain metabolism between *ent*-PREGS and PREGS.

### Example 9 : In vivo metabolism of PREGS and ent-PREGS in rat: steroid sulfatase activity

The metabolism of PREGS and *ent*-PREGS was investigated 5 min after ICV administration of 1µg of each compound in rat and the unconjugated PREG was quantified in the brain by gas-chromatography-mass spectrometry (Liere et al., 2000, *J. Chromatogr. B Biomed Sci. Appl.* 2000,739:301-12).

Compared with the saline-injected control rats, the brain content of PREG was signicantly increased after PREGS injection.

In contrast, the amount of PREG in the brain did not significantly differ between *ent*-PREGS-treated rats and the controls.

Thus, these data revealed differences in brain metabolism between ent-PREGS and PREGS. Remarkably, they show that *ent*-PREGS is not metabolized by sulfatase *in vivo.*

In addition, they strongly suggest that the steroidogenic pathway leading to steroid homones does not occur after administration of certain steroid enantiomers, including *ent*-PREGS.

### Example 10 : In vitro metabolism of PREGS and ent-PREGS in rat: steroid sulfatase activity by brain and liver microsomes

The metabolism of PREGS and *ent*-PREGS was examined after incubation of 8 µg of each steroid with 200 µg microsomal proteins prepared from rat brain or liver, at 37°C for lhour, and the unconjugated PREG was quantified in the brain by gas-chromatography-mass spectrometry (Liere et al., 2000, *J. Chromatogr. B Biomed. Sci. Appl.* 2000,739:301-12)

PREGS was found to metabolized into PREG in both tissues, the levels of PREG being 4 times higher in the liver than in the brain

Remarkably, the formation of PREG was undectectable when *ent*-PREGS was used as a substrate, during the incubations with brain or liver microsomes.

Thus these data show ent-PREGS is not metabolized by sulfatases in vitro and confirms the differences in metabolism between *ent*-PREGS and PREGS observed *in vivo* (Example 9).

### Example 11 : In vitro metabolism of DHEAS and ent-DHEAS in rat: steroid sulfatase activity by brain and liver microsomes

The metabolism of DHEAS and *ent*-DHEAS was examined after incubation of 8 µg of each steroid with 200 µg microsomal proteins prepared from rat brain or liver, at 37°C for 1hour, and the unconjugated DHEA was quantified in the brain by gas-chromatography-mass spectrometry (Liere et al., 2000, *J. Chromatogr. B Biomed. Sci. Appl.* 2000,739:301-12)

DHEAS was found to metabolized into DHEA in both tissues, the levels of DHEA being 5 times higher in the liver than in the brain

Remarkably, the formation of DHEA was undectectable when *ent*-DHEAS was used as a substrate, during the incubations with brain or liver microsomes.

Thus, these results indicate that *ent*-DHEAS is not metabolized by sulfatases *in vitro.*

They also suggest that the steroidogenic pathway leading to steroid homones does not occur after administration of certain steroid enantiomers, including *ent*-DHEAS.

Throughout this application, various publications, patents and published patent applications are cited. The disclosures of these publications, patents and published patent specifications referenced in this application are hereby incorporated by reference into the present disclosure.

## Claims

1. A pharmaceutical composition comprising an enantiomer of a steroid and a pharmaceutically acceptable carrier.

2. The pharmaceutical composition of claim 1, wherein said steroid is a neurosteroid.

3. The pharmaceutical composition of claim 2, wherein said neurosteroid is DHEA sulfate.

4. The use of an enantiomer of a steroid for the preparation of a medicament that is effective in the restoration of memory loss in humans.

5. The use of claim 4, wherein said memory loss is an age-related decline in memory function, dementia, amnesia, or is the result of a neurological disorder, a neuropsychiatric disorder, or a neurodegenerative disease such as Alzheimer's Disease.

6. The use of claim 4, wherein said steroid is a neurosteroid.

7. The use of claim 4, wherein said steroid is a steroid hormone.

8. The use of claim 4, wherein said steroid is modified at least at one of the chiral or non chiral centers.

9. The use of claim 8, wherein said steroid is modified by hydoxylation, esterification, etherification, reduction, oxididation, or substitution with an halogen or an alkyl group.

10. The use of claim 4, wherein said steroid is DHEA sulfate.
